# EUROPEAN PATENT SPECIFICATION

(11) **EP 1 783 489 B1**
(45) Date of publication and mention of the grant of the patent: **17.04.2013**
(21) Application number: 05765433.7
(22) Date of filing: 29.06.2005
(51) Int. Cl.: C12N 5/16, G01N 33/569

(54) **METHOD OF SEPARATING SMALL HEPATOCYTES BY USING SPECIFIC ANTIBODY**
VERFAHREN ZUR TRENNUNG KLEINER HEPATOZYTEN UNTER VERWENDUNG EINES SPEZIFISCHEN ANTIKÖRPERS
PROCEDE DE SEPARATION DE PETITS HEPATOCYTES METTANT EN OEUVRE UN ANTICORPS SPECIFIQUE

(30) Priority: 29.06.2004 JP 2004191476
(43) Date of publication of application: 09.05.2007
(73) Proprietor: LSIP, LLC, Chiyoda-ku Tokyo 100-0005 (JP)
(72) Inventor: MITAKA, Toshihiro, Sapporo-shi, Hokkaido 0040863 (JP); KON, Junko, Sapporo Medical University, Sapporo-shi, Hokkaido 0608556 (JP)
(74) Representative: Cabinet Plasseraud
(86) International application number: PCT/JP2005/011914
(87) International publication number: WO 2006/001472

(56) References cited:
- EP-A- 1 291 415
- JP-A- 9 313 172
- DATABASE MEDLINE [Online] US NATIONAL LIBRARY OF MEDICINE (NLM), BETHESDA, MD, US; December 2004 (2004-12), ZHAO XUN-ZHUAN ET AL: "[Isolation, culture and multipotent differentiation of mesenchymal stem cells from human fetal livers]" XP002474875 Database accession no. NLM15619334 & ZHONGHUA GAN ZANG BING ZA ZHI = ZHONGHUA GANZANGBING ZAZHI = CHINESE JOURNAL OF HEPATOLOGY DEC 2004, vol. 12, no. 12, December 2004 (2004-12), pages 711-713, ISSN: 1007-3418
- LAZARO ET AL: "Establishment, characterization, and long-term maintenance of cultures of human fetal hepatocytes" HEPATOLOGY, WILLIAMS AND WILKINS, BALTIMORE, MD, US, vol. 38, no. 5, November 2003 (2003-11), pages 1095-1106, XP005293584 ISSN: 0270-9139
- OGAWA K ET AL: "THE GENERATION OF FUNCTIONALLY DIFFERENTIATED, THREE-DIMENSIONAL HEAPTIC TISSUE FROM TWO-DIMENSIONALSHEETS OF PROGENITOR SMALL HEPATOCYTES AND NONPARENCHYCAL CELLS" TRANSPLANTATION, WILLIAMS AND WILKINS, BALTIMORE, MD, US, vol. 77, no. 12, 27 June 2004 (2004-06-27), pages 1783-1789, XP002459230 ISSN: 0041-1337
- TATENO C ET AL: "GROWTH AND DIFFERENTIATION IN CULTURE OF CLONOGENIC HEPATOCYTES THAT EXPRESS BOTH PHENOTYPES OF HEPATOCYTES AND BILIARY EPITHELIAL CELLS" AMERICAN JOURNAL OF PATHOLOGY, PHILADELPHIA, PA, US, vol. 149, no. 5, November 1996 (1996-11), pages 1593-1605, XP000995580 ISSN: 0002-9440
- CHO M K ET AL: "Hyaluronic acid inhibits adhesion of hepatic stellate cells in spite of its stimulation of DNA synthesis" TISSUE AND CELL, CHURCHILL LIVINGSTONE MEDICAL JOURNALS, EDINBURGH, GB, vol. 36, no. 5, October 2004 (2004-10), pages 293-305, XP004607721 ISSN: 0040-8166
- KON J. ET AL: 'Kogata Kansaibo Tokuiteki Hyomen Marker no Hatsugen to Kotai ni yoru Saibo Bunri' SAISEI IRYO vol. 4, SPECIAL EXTRA ISSUE, 10 February 2005, page 105, WS-10-4, XP002998939
- HERLEVSEN M. ET AL: 'The association of the tetraspanin D6.1A with the alpha6beta4 integrin supports cell motility and liver metastasis formation' J.CELL SCI. vol. 116, no. 21, 2003, pages 4373 - 4390, XP002998940
- WU H. ET AL: 'bri3, a novel gene, participates in tumor necrosis factor-alpha-induced cell death' BIOCHEM.BIOPHYS.RES.COMMUN. vol. 311, no. 2, 2003, pages 518 - 524, XP002300188
- VIDAL R. ET AL: 'Sequence, genomic structure and tissue expression of Human BRI3, a member of the BRI gene family' GENE vol. 266, no. 1-2, 2001, pages 95 - 102, XP004233097
- SCHMIDT D.S. ET AL: 'CD44 variant isoforms associate with tetraspanins and EpCAM' EXP.CELL RES. vol. 297, no. 2, 15 July 2004, pages 329 - 347, XP002998941

### SEQUENCE LISTING

<110> Japan Science and Technology Agency
<120> Methods of isolating small hepatocytes with specific antibodies
<130> Y1M0395
<150> JP 2004-191476
   <151> 2004-06-29
<160> 13
<170> PatentIn version 3.2
<210> 1
   <211> 29
   <212> DNA
   <213> Artificial
<220>
   <223> PCR primer
<400> 1
   cccgaattca tggacaaggt ttggtggca 29
<210> 2
   <211> 29
   <212> DNA
   <213> Artificial
<220>
   <223> PCR primer
<400> 2
   cccgaattcc tacaccccaa tcttcatat 29
<210> 3
   <211> 29
   <212> DNA
   <213> Artificial
<220>
   <223> PCR primer
<400> 3
   cccgaattca tggcaggtgt cagtggctg 29
<210> 4
   <211> 29
   <212> DNA
   <213> Artificial
<220>
   <223> PCR primer
<400> 4
   cccgaattct catttgcttc caatttggc 29
<210> 5
   <211> 29
   <212> DNA
   <213> Artificial
<220>
   <223> PCR primer
<400> 5
   cccgaattca tggtgaagat cagtttcca 29
<210> 6
   <211> 29
   <212> DNA
   <213> Artificial
<220>
   <223> PCR primer
<400> 6
   cccgaattct cacaccaccc cacagatga 29
<210> 7
   <211> 20
   <212> DNA
   <213> Artificial
<220>
   <223> PCR primer
<400> 7
   accacagtcc atgccatcac 20
<210> 8
   <211> 20
   <212> DNA
   <213> Artificial
<220>
   <223> PCR primer
<400> 8
   tccaccaccc tgttgctgta 20
<210> 9
   <211> 1095
   <212> DNA
   <213> Rottus norvegicus
<400> 9
   atggacaagg tttggtggca cacagcttgg ggactacttt gcctcttaca gttgagcctg 60
   gcacagcagc agatcgattt gaatataacc tgccgttacg caggtgtatt ccatgtggag 120
   aaaaatggcc gctacagtat ctccaggact gaagcagctg acctctgcga ggctttcaac 180
   accaccttgc ccaccatggc tcagatggag ttagccctga gaaaggggtt tgaaacatgc 240
   aggtatgggt tcatagaagg acacgtggta atcccgagga tccaccccaa cgctatctgt 300
   gcagccaaca acacaggagt gtatatcctc ctcgcatcca acacctccca ctatgacaca 360
   tattgcttca atgcctcagc tcctcttgaa gaagactgta catcagtcac agacctaccc 420
   aattccttcg atggaccagt taccataact attgtcaacc gtgatggcac ccgctacagc 480
   aagaagggcg agtatagaac acaccaagaa gacatcgatg cctcaaacat tatagatgag 540
   gatgtcagca gtggatccac cattgagaag agcaccccag aaggctacat tttgcacacc 600
   gaccttccca cttcacagcc tactggagac cgggatgacg ccttctttat tgggagcacc 660
   ctggccacca gtgatggaga ctcatccatg gaccccaggg gtggtttcga cactgtgact 720
   catggatccg aattagctgg acactcaagt gggaatcaag acagtggagt gaccacaact 780
   tctggtcctg cgaggagacc tcagattcca gagtggctta tcatcttggc atccctcctg 840
   gcgctggctc tgattcttgc cgtctgcatt gctgtcaaca gtaggagaag gtgtgggcag 900
   aagaagaagc tggtgatcaa cagtggcaat ggaacagtgg aagacaggaa accaagtgaa 960
   ctcaacgggg aggccagcaa gtctcaggaa atggtgcatt tggtgaacaa ggaaccaaca 1020
   gagactccgg accagtttat gacagctgat gagacccgga atctgcagag tgtggatatg 1080
   aagattgggg tgtag 1095
<210> 10
   <211> 708
   <212> DNA
   <213> Rottus norvegicus
<400> 10
   atggcaggtg tcagtggctg tttaaagtat tcgatgtttt tcttcaactt cttgttctgg 60
   gtatgtggta cactgatcct gggtctagcc atatggttga gagtaagcaa agacggcaaa     120
   gagattatca catccggtga caatggcacg aaccccttca ttgctgtgaa tatcttgatt     180
   gcagttgggt ccatcatcat ggttctgggt ttcctgggat gctgcggagc tgtgaaagaa 240
   agtcgctgca tgcttctctt gttttttatc ggcttgctcc tgattctgct tctgcaagta 300
   gccgcaggta tcctaggagc tactttcaaa tctgaatcta gccgcattct gaatgaaacg 360
   ctctatgaaa atgccaagct tttgtcagaa acaagcaatg aggcaaaaga agtacagaag 420
   gccatgattg catttcagtc agagtttaag tgctgtggct tgagatttgg agctgctgac 480
   tggggaaaaa attttccaga tgcgaaagag tcatgccaat gtacaggatc tgattgtgaa 540
   agttataatg gagagaatgt ctatcgaacg acctgtcttt ctctgataaa agagctggtt 600
   gaaaaaaaca ttattattgt cattggaatt gcctttggac tggcagttat cgagattctt 660
   ggtttggtgt tttctatggt gctctactgc caaattggaa gcaaatga 708
<210> 11
   <211> 840
   <212> DNA
   <213> Rottus norvegicus
<400> 11
   atggtgaaga tcagcttcca gcccgcggta gcgggcgtca aggccgaaaa agcagacaaa 60
   gcagcggcgt ctggtccggc ctcggcatct gccccggccg cggagattct gctgacgccg    120
   gcgagggagg aacgcccccc tcgccaccgc tccaggaaag ggggctccgt gggcggtgtg    180
   tgctacctgt ccatgggcat ggttgtactg cttatgggcc ttgtgtttgc ctcggtctac    240
   atctacagat acttcttcct tgctcagctg gcccgcgata acttcttcca ctgcggtgtg    300
   ctctacgaag actccctatc ctcccagatc cggactcggc tggaactgga ggaggatgtg    360
   aagatctatc ttgaggagaa ctatgaacgt attaatgtcc ctgtgcccca gtttggtggt    420
   ggggaccctg cagacatcat ccatgacttt cagcggggtc tcactgccta ccacgacatc    480
   tccctggaca agtgctacgt cattgagctc aacaccacca tcgtgctgcc cccacgaaac    540
   ttctgggagc tcctcatgaa cgtgaagaga gggacctacc tcccacagac gtacatcatc    600
   caggaggaga tggtggtgac agagcatgtc cgagacaagg aggctctggg ttcctttatc    660
   taccacctgt gcaacgggaa ggacacctat cggctgcggc gccgggccac ccggaggcgg    720
   atcaacaagc gtggggccaa gaactgcaat gccatccgcc acttcgagaa tacatttgtg    780
   gtggagacgc tcatctgtgg ggtggtgtga gtggagacgc tcatctgtgg ggtggtgtga    840
<210> 12
   <211> 17
   <212> PRT
   <213> Rattus norvegicus
<400> 12
   Phe Gly Ala Ala Asp Trp Gly Lys Asn Phe Pro Asp Ala Lys Glu Ser
   1               5                   10                  15
   Cys
<210> 13
   <211> 20
   <212> PRT
   <213> Rattus norvegicus
<400> 13
   Leu Thr Pro Ala Arg Glu Glu Arg Pro Pro Arg His Arg Ser Arg Lys
   1               5                   10                  15
   Gly Gly Ser Val
               20

## Description

### Field of the Invention

The present invention relates to a method of efficiently isolating proliferative hepatocytes (small hepatocytes) derived from mammalian including human.

### Background of the Invention

Liver and hepatocytes possess multiple functions so that they are compared to a chemical factory in the living body. For example, more than 90% of serum proteins are produced by hepatocytes, which have a detoxification function of metabolizing toxic substances that were incorporated into or produced in the living body. Thus researchers in various institutions have been attempted to culture hepatocytes, detect harmful substances (as a bio-sensor) and allow to produce substances needed for human being in vitro by using the functions of hepatocytes. In order to supply hepatocytes used for such investigations, it is necessary to isolate mature hepatocytes for every experiment, because there are currently no cell lines possessing the differentiated functions of hepatocytes. In such circumstances, the number of obtainable cells depends on the number of hepatocytes in individuals, because a method of proliferating mature hepatocytes with the differentiated functions has not been established. Therefore, a continuous supply of a large number of cells having most of the functions of mature hepatocyte has been desired in the development of built-in type artificial livers. It has been an important issue to develop a method of cryopreserving hepatocytes, preserving them for a long time, and reusing hepatocytes from animals including human being, which has been investigated worldwide. However, there are previously only a few reports wherein cryopreserved hepatocytes had likely adhered to the culture dishes after being thawed and had been able to maintain about 70 to 80% of hepatic functions only for a short period. Furthermore, in those reports, the thawed hepatocytes could not proliferate and died within a short period.

Hepatic dysfunction of human beings is caused by various diseases such as hepatitis, hepatic cirrhosis and liver tumors. Because artificial liver has not been yet in the practical stage, the liver transplantation is only the ultimate treatment of such diseases. In addition, though there are numerous patients who require the liver transplantation of the liver not only in Japan but also in many countries in the world, the number of the donors for donating the organ is only 10 % of the required number. Thus, it is demanded to develop a process for producing a liver tissue *in vitro* which is usable for liver transplantation, a process for preparing colonies of precursor cells for liver tissue usable for the process and a process for producing them *in vitro.*

On the other hand, the inventors reported a cell population in the liver tissue which exhibited similar phenotypes for markers of mature hepatocytes such as albumin, transferrin, cytokeratin (CK) 8 or CK18 and had the ultrastructural characteristics for mature hepatocyte, while the population was composed of small cells having high proliferation ability. These cells -were named "small hepatocyte" (Mitaka T. et al., Hepatology, 16, 440-447 (1992), Mitaka T., Sato F, Mizuguchi T.et al., Hepatology 29, 111-135 (1999)).

Additionally, there are several reports on small hepatocytes and hepatic parenchymal cells having proliferation capacity (Japanese Patent No. 3211941, Laid Open Japanese Patent Application (JP-Kokai) No.2002-078481, JP-Kokai No.09-313172, JP-Kokai No. 08-112092). JP-Kokai No.2002-045087 also describes a chimeric animal having hepatocytes derived from a heterologous animal including human and a test method using the chimeric animal.

The inventors also reported a method of obtaining a fraction enriched with small hepatocytes (WO 01/92481), a method of cryopreservation of small hepatocytes retaining the liver function and proliferation capacity and a method of preparing small hepatocytes suitable for such methods (WO 01/92481). The inventors further reported a colony of small hepatocytes suitable for preparing a transplantable liver tissue, a method of preparing thereof, a method of inducing a liver tissue from small hepatocytes (WO 02/088332) and a method of estimating the effects of drugs *in vitro,* particularly the effects associated with normal liver functions. However, a marker protein which is specific for the mammalian (including human) proliferative hepatocytes (small hepatocytes) has not been clearly determined, and therefore there is place left for improving an efficient method of isolating the cells.

### Summary of the Invention

The present invention provides an efficient method of isolating mammalian (including human) proliferative hepatocytes (small hepatocytes).

The inventors determined three transmembrane proteins which are specifically expressed on small hepatocytes and found that the small hepatocytes could be efficiently isolated by using antibodies against these specific proteins.
1) The present invention provides a method of isolating small hepatocytes, comprising the steps of
   i) contacting a cell suspension containing cells from a liver of a mammalian with one or more antibody selected from the group consisting of an antibody against CD44 (anti-CD44 antibody), an antibody against D6.1A (anti-D6.1A antibody) and an antibody against BR13 (anti-BRI3 antibody) to form an immunocomplex containing the small hepatocytes which was contained in the cell suspension and the one or more antibody; and,
   ii) recovering the immunocomplex containing the small hepatocytes.
      More particularly, the present invention is also the above-specified method wherein the one or more antibody selected from the group consisting of anti-CD44 antibody, anti-D6.1A antibody and anti-BRI3 antibody is labeled with biotin and the step of recovering the immunocomplex comprises contacting the immunocomplex and a carrier which is linked to avidin and/or avidin labeled with a fluorochrome.
      The method may optionally include the steps of
   iii) releasing the small hepatocytes from the immunocomplex; and,
   iv) recovering the released small hepatocytes.
2) The present invention also provides a method of isolating small hepatocytes, comprising the steps of
   i) contacting a cell suspension containing cells from a liver of a mammalian with one or more antibody selected from the group consisting of anti-CD44 antibody, anti-D6.1A antibody and anti-BRI3 antibody to form a first immunocomplex containing the small hepatocytes which was contained in the cell suspension and the one or more antibody;
   ii) contacting the first immunocomplex with one or more antibody which binds to at least one of the antibodies used in step i) selected from the group consisting of anti-CD44 antibody, anti-D6.1A antibody and anti-BR13 antibody to form a second immunocomplex; and,
   iii) recovering the second immunocomplex containing the small hepatocytes.
      The method may optionally include the steps of
   iv) releasing the small hepatocytes from the second immunocomplex; and,
   v) recovering the released small hepatocytes.
3) The present invention also provides a method of isolating small hepatocytes, comprising the steps of
   i) contacting one or more of antibodies selected from the group consisting of anti-CD44 antibody, anti-D6.1A antibody, anti-BRI3 antibody with one or more antibody which binds to at least the one or more of antibodies selected from the group consisting of anti-CD44 antibody, anti-D6.1A antibody, anti-BRI3 antibody to form a first immunocomplex;
   ii) contacting the first immunocomplex with a cell suspension containing cells from a liver of a mammalian to form a second immunocomplex; and
   iii) recovering the second immunocomplex containing the small hepatocytes.
      The method may optionally include the steps of
   iv) releasing the small hepatocytes from the second immunocomplex; and,
   v) recovering the released small hepatocytes.
4) The present invention also provide a method of isolating small hepatocytes, comprising the steps of
   i) contacting a cell suspension containing cells from a liver of a mammalian with one or more antibody selected from the group consisting of anti-CD44 antibody, anti-D6.1A antibody and anti-BRl3 antibody to form a first immunocomplex containing the small hepatocytes which was contained in the cell suspension and the one or more antibody, wherein the one or more antibody is linked to a carrier; and,
   ii) recovering the immunocomplex containing the small hepatocytes.
      The method may optionally include the steps of
   iii) releasing the small hepatocytes from the immunocomplex; and,
   iv) recovering the released small hepatocytes.
5) The present invention also provides a method of isolating small hepatocytes comprising the steps of
   i) contacting a cell suspension containing cells from a liver of a mammalian with one or more antibody selected from the group consisting of anti-CD44 antibody, anti-D6.1A antibody and anti-BRI3 antibody to form a first immunocomplex containing the small hepatocytes which was contained in the cell suspension and the one or more antibody;
   ii) contacting the first immunocomplex with one or more antibody which binds to at least one of the antibodies used in step i) selected from the group consisting of anti-CD44 antibody, anti-D6.1A antibody and anti-BR13 antibody to form a second immunocomplex, wherein the at least one of the antibodies used in step i) is linked to a carrier; and,
   iii) recovering the second immunocomplex containing the small hepatocytes.
      The method may optionally include the steps of
   iv) releasing the small hepatocytes from the second immunocomplex; and,
   v) recovering the released small hepatocytes.
6) The present invention also provides a method of isolating small hepatocytes comprising the steps of
   i) contacting one or more of antibodies selected from the group consisting of anti-CD44 antibody, anti-D6.1A antibody, anti-BRI3 with one or more antibody which binds to at least the one or more of antibodies selected from the group consisting of anti-CD44 antibody, anti-D6.1A antibody and anti-BRI3 to form a first immunocomplex, wherein the one or more antibody which binds to at least one of the one or more of antibodies selected from the group consisting of anti-CD44 antibody, anti-D6.1A antibody and anti-BRI3 is linked to a carrier;
   ii) contacting the first immunocomplex with a cell suspension containing cells from a liver of a mammalian to form a second immunocomplex; and
   iii) recovering the second immunocomplex containing the small hepatocytes.
      The method may optionally include the steps of
   iv) releasing the small hepatocytes from the second immunocomplex; and,
   v) recovering the released small hepatocytes.
      A method is also provided where in each of the above-specified method the cell suspension is derived from a liver of a mammalian which has received a galactosamine or the like.

### Brief Description of the Drawings

Figure 1 shows the results of analysis of mRNA expression levels of CD44, D6.1A and BRI3 at the time of preparation and at each indicated time after starting culture, as determined by using RT-PCR. (A): CD44, (B): D6.1A, (C): BR13. The longitudinal axes indicate the relative intensity and the horizontal axes indicate the days of culture.
Figure 2 shows the comparison of mRNA expression levels of CD44, D6.1A and BRI3 in small hepatocytes and mature hepatocytes, as determined by RT-PCR. SH: small hepatocytes, MH: mature hepatocytes. The longitudinal axis indicates the relative intensity.
Figure 3 shows the results of comparison of mRNA expression levels of CD44, D6.1A and BRI3 in small hepatocytes and mature hepatocytes, as determined by Northern blot. SH: small hepatocytes, MH: mature hepatocytes. The longitudinal axis indicates the relative intensity. Upper represents the longer mRNA among detected mRNAs and Lower represents the smaller mRNA among detected mRNAs in the small hepatocytes.
Figure 4 shows the CD44 expression in small hepatocytes which had been induced to maturation by Matrigel, as determined by Northern Blot. SH: small hepatocytes, MH: mature hepatocytes. The longitudinal axis indicates the relative intensity.

### Description of the Preferred Embodiments

The present invention is a method of efficiently isolating small hepatocytes. The term "small hepatocyte" as used herein does not merely mean a small cell which is derived from a liver, but it means a small cell which may be isolated according to the following procedures or the similar procedures and which exhibits substantially similar phenotypes to a mature hepatocyte regarding the markers such as albumin, transferrin, cytokeratin (CK) 8 and CK18 and has the ultrastructural features as a hepatocyte. These cells were found by the inventors, the details of which were described in Mitaka, T. et al., Hepatology, 16, 440-447 (1992) and Mitaka T., Sato F, Mizuguchi T. et. al., Hepatology 29, 111-135 (1999).

A fraction enriched with the small hepatocytes may be prepared as follows. Liver-derived cells can be obtained by treating liver tissue from human being or other animals with a solution containing a collagenase and the like. For this purpose, a conventional liver perfusion method may be used. The obtained cell suspension may be passed through a mesh of an appropriate size to remove tissue debris such as undigested tissue residues and the like, if necessary. Although the cell suspension can be directly used to separate the small hepatocytes according to the method of the present invention, it is preferred to remove parenchymal cells and undesired tissue residues to a certain extent before applying the method of the present invention on them.

Parenchymal cells can be removed by a low speed centrifugation as described below. The low speed centrifugation is the condition which is sufficient to separate the light fraction which is enriched with nonparenchymal cells from the fraction mainly containing parenchymal cells and undesired tissue residues, preferably the condition sufficient to separate the light fraction which is enriched with small hepatocytes and nonparenchymal cells and which is substantially free of parenchymal cells from the fraction mainly containing parenchymal cells and undesired tissue residues. When the cells obtained from a liver according to the described method are fractionated under such conditions, the small hepatocytes may be obtained in the light fraction in larger quantities. Particularly, the cell suspension can be separated into a heavy fraction which mainly contains parenchymal cells and a light supernatant fraction which mainly contains relatively light cells including non-parenchymal cells such as stellate cells, Kupffer cells and sinusoidal endothelial cells by, for example, centrifuging at 50 x g for one (1) minute. Larger amount of the small hepatocytes may be obtained in the supernatant fraction under this centrifugation condition.

The centrifugation is preferably performed at about 50 x g for not more than 1 minute, because the ratio of precipitated small hepatocytes may increase as the speed or the time of centrifugation increases, although the ratio of parenchymal cells in the supernatant fraction decreases. Parenchymal cells and undesired tissue debris and the like may be further removed by repeatedly centrifuging, precipitating and suspending the supernatant fraction. More specifically, for example, the supernatant fraction may be centrifuged at 50 x g for 5 minutes and the precipitate is suspended in an appropriate medium and further centrifuged at 50 x g for 5 minutes. The precipitate is suspended in a similar medium, centrifuged again at 50 x g for 5 minutes. The precipitate is suspended in a similar medium and centrifuged at 150 x g for 5 minutes and the precipitated cells are suspended in a fresh medium. The number of cells in the cell suspension may be counted to adjust the cell density required for the subsequent culture or the treatments. Generally, the density is adjusted to 1 x 10⁴ to 5 x 10⁵ cells/ml.

Small hepatocytes may be efficiently isolated from such prepared fraction containing a large amount of small hepatocytes according to the method of present invention. Small hepatocytes may also be efficiently isolated according to the method of present invention after culturing such prepared fraction enriched with small hepatocytes for a certain period (pre-culture) to proliferate the small hepatocytes. The pre-culture is preferably performed in the presence of a carrier to which hyaluronic acid has attached (hyaluronic acid-attached carrier) or a carrier of which a major component is hyaluronic acid (hyaluronic acid-based carrier).

It was revealed that the expression of CD44 is likely to be distinguishing from day 2 to 3 of primary culture and reached to nearly a maximum at day 3 to 5 of the culture. For D6.1A, it was revealed that it could be detected from about day 3 of primary culture and reached to nearly a maximum at about day 18 of the culture. Additionally, for BRI3, it was revealed that it was distinguishing at about day 2 of primary culture and reached to nearly a maximum after 5 to 11 days of culture. Thus, the inventors contemplated the possibility that it would take 2 to 3 days for activating the small hepatocytes which has potentially existed, because the cell may be damaged by preparing the cell fraction from a liver. Additionally, the inventors confirmed that when a rat received a sever damage by galactosamine or the like, CD44-positive cells appeared in the liver tissue after a few days from administrating galactosamine or the like, which is consistent with the aforementioned contemplation. Therefore, when the cell fraction is prepared from a liver of an individual which has received a severe damage by galactosamine or the like, small hepatocytes may be very efficiently isolated by a pre-culture of short period or without a pre-culture.

For damaging a rat with galactosamine or the like, a rat will receive D-galactosamine at a concentration of 10 mg to 100 mg of D-galactosamine (Across) in 200 µl PBS per 100 g body weight by intraperitoneal injection. Cells may be dissociated from the liver at days 3 to 6 post-injection according to the method of Seglen (Selgen, PO., Methods Cell Biol., 1976, 13, 29-83).

When no such damage by galactosamine is caused, a pre-culture of 3 to 18 days, preferably 3 to 5 days is favored. If the culture is performed in the presence of a hyaluronic acid-attached carrier such as a culture dish to which hyaluronic acid has attached, a porous material to which hyaluronic acid has attached, a carrier made of hyaluronic acid or a hyaluronic acid-based carrier, the culture period may be longer, for example, the period of pre-culture may be 3 to 21 days. When the culture is conducted in the presence of hyaluronic acid, the small hepatocytes may be isolated more efficiently after culturing a cell population which is expected to contain small hepatocytes in the presence of hyaluronic acid, because small hepatocytes selectively proliferate in the presence of hyaluronic acid.

When the pre-culture is conducted in the presence of hyaluronic acid, a hyaluronic acid-attached carrier may be prepared as follows. Hyaluronic acid may be prepared as a solution in an appropriate medium or buffer at a suitable concentration such that when 1 ml to 5 ml of the prepared solution is contacted with a carrier the amount of hyaluronic acid will be 10 µg/cm² to 1000 µg/cm² preferably 50 µg/cm² to 500 µg/cm² based on the surface area of the carrier. In general, the solution of hyaluronic acid will be conveniently prepared as a stock solution in an appropriate medium or buffer at a concentration of 10-20 mg/ml. The buffer used can be any buffer as long as it is not harmful for cells, including Hank's solution, a traditional cell culture medium, saline, phosphate-buffered saline (PBS) or the like. The pH of the buffer may be within the range where hyaluronic acid can be dissolved, preferably 7.1 to 8.5. The temperature for dissolving hyaluronic acid may be any temperature as long as hyaluronic acid can be dissolved and does not decompose. It would be convenient to use 30°C to 40°C, preferably about 37°C, considering the temperature setting of an ordinary incubator.

In this connection, hyaluronic acid may be generally classified into low molecular weight hyaluronic acid and high molecular weight hyaluronic acid by setting a demarcation at molecular weight of about one million. The high molecular weight hyaluronic acid is preferable when the pre-culture is conducted in the presence of hyaluronic acid.

Thus prepared hyaluronic acid solution may be combined directly or optionally after diluting it with an appropriate buffer or medium together with a carrier such as described above to coat hyaluronic acid on the carrier. When a relatively water-repellent carrier is to be coated, it is particularly useful to increase the volume of the hyaluronic acid solution by dilution because the surface of the carrier may not be sufficiently covered by the hyaluronic acid solution if the volume of the solution for coating is small. For coating the carrier, hyaluronic acid solution of 0.5-20 mg/ml, preferably 1-10 mg/ml is contacted with the surface of the carrier at 10 µg to 1000 µg, preferably 50 µg to 500 µg per 1 cm² of the surface area of the carrier. For example, when a 60mm-dish is to be coated with hyaluronic acid, a solution of hyaluronic acid prepared at a concentration of 10 mg/ml will be diluted to 1 mg/ml by PBS or the like and 1 ml of the dilute will be poured to the dish to coat it. The contact of the hyaluronic acid solution with the carrier is conducted by incubating them at 30°C to 40°C, particularly about 37°C for one to 24 hours. The temperature and period for incubation may be those sufficient for the hyaluronic acid to be coated on the carrier. The incubation period may be longer than 24 hours, but there would be less merit of long-term incubation.

When pre-culture is conducted, the fraction (cell population) which was prepared as described above and is enriched with small hepatocytes may be cultured in a basal medium containing serum, nicotinamide, vitamin C, antibiotics, growth factors and other additives commonly used for cell culture, for example Dulbecco's modified Eagle's medium supplemented with these substances, at 37°C. The medium for proliferating or maintaining the small hepatocytes preferably contains nicotinamide, vitamin C, growth factors, DMSO and the like. Vitamin C is usually added as ascorbic acid 2-phosphate preferably at a concentration of 0.1 mM to 1.0 mM, more preferably 0.5 to 1.0 mM, and nicotinamide is used at a relative high concentration, preferably 1 to 20 mM and more preferably 5 to 10 mM. As the growth factors, epidermal growth factor (EGF), hepatocyte growth factor (HGF), or transforming growth factor α (TGF α) and the like may be used. TGF α is particularly preferred. When TGF α is added, it is preferably to use it at a concentration of 1 µg/l to 100 µg/l, more preferably 5 µg/l to 50 µg/l.

In the primary culture, DMSO is preferably added at a concentration of about 0.1 to about 2 % (v/v), more preferably about 0.5 % to about 1.5% (v/v) on and after day 4 of the culture. Additionally, as described above, the pre-culture may be conducted in the presence of hyaluronic acid, such as hyaluronic-attached carrier, a carrier made of hyaluronic acid, or a hyaluronic acid-based carrier.

For the container for the pre-culture, any culture dish conventionally used for cell culture may be used. Generally, collagen-coated dishes are used for the culture of adherent cells. For example, the culture dishes of various sizes which are coated by collagen derived from bovine dermis or rat tail are commercially available. It is also possible to prepare such dishes, if necessary, but it is preferable to use dishes which are not coated by collagen because the cells can be dissociated from the dishes in the mild condition more easily when the extracellular matrix is less. If pre-cultured, the culture may be disaggregated into individual cells in the presence of metal chelators and/or enzymes and the cells can be successively subcultured to re-form colonies. However, in this case, since the damages to the cells may be extensive, it is preferable to dissociate the colonies from culture containers by non-enzymatic procedures. The metal chelators which may be used are any ones as long as their cytotoxicity is less, for example EDTA, EGTA and/or the salts thereof may be used at the concentration generally used for them. Among non-enzymatic dissociation agents, a solution of Hank's buffer (pH 7.3 - 7.5) without Ca and Mg supplemented with EDTA, glycerol, sodium citrate and the like may be used. For example, a prepared non-enzymatic cell dissociation agent can be commercially available from Sigma Chemical Co., which is marketed as "Cell Dissociation Solution". For the culture, a conventional 5 % CO₂ incubator may be used. The range of CO₂ concentration and the temperature are not essential as long as they are within the range which is acceptable for conventional cultured cells. Specifically, a cell suspension enriched with pre-cultured small hepatocytes can be obtained by using the methods and conditions described in WO 01/92481.

The cell suspension prepared as described above or the cell suspensions enriched with small hepatocytes which has further been pre-cultured may be contacted with one or more antibody selected from the group consisting of an antibody against CD44 (anti-CD44 antibody), an antibody against D6.1A (anti-D6.1A antibody) and an antibody against BRI3 (anti-BRI3 antibody) to form an immunocomplex containing the small hepatocytes contained in the cell suspension and the one or more antibody. The antibody may be labeled or may be linked to a carrier. Alternately, the cell suspension enriched with small hepatocytes with one or more antibody (primary antibody(ies)) selected from the group consisting of anti-CD44 antibody, anti-D6.1A antibody and anti-BRI3 antibody to form a first immunocomplex, and then contacting the first immunocomplex with one or more antibody (secondary antibody(ies)) which binds to at least one of the used antibodies selected from the group consisting of anti-CD44 antibody, anti-D6.1A antibody and anti-BRI3 antibody to form a second immunocomplex. In this case, one or more of the antibodies (primary antibody(ies)) selected from the group consisting of anti-CD44 antibody, anti-D6.1A antibody, anti-BRI3 antibody is firstly contacted with one or more antibody (secondary antibody(ies)) which binds to at least one of the primary antibodies to form a first immunocomplex, and then the first immunocomplex may be contacted with a cell suspension containing cells from a liver of a mammalian to form a second immunocomplex. In any cases, the secondary antibody may be labeled or may be linked to a carrier.

Release of the antibody from the immunocomplex may be performed according to a method well known to those skilled in the art. The small hepatocytes forming the immunocomplex may be further cultured as is, because the bound antibodies will be diluted as the cells proliferate. When the antibody is linked to a carrier, the carrier-antibody linkage may be cleaved by enzyme digestion or other method depending on the linkage mode. Such methods are well known to those skilled in the art, and commercially available agents may be used as described below. For example, the Fc portion of the antibody may be cleaved by papain or pepsin or the like. Particularly, when a label or a carrier is linked to the Fc portion of the antibody, the label or the carrier can be cleaved from the cells by such enzymes. Additionally, when a particular antibody is used, which is obtained from a sheep immunized with the F(ab)₂ fragment of the antibody which was directly used for isolating the small hepatocytes, it will interact with the binding of the antigen determinant of the bound antibody (for example, anti-CD44 antibody, anti-D6.1 A antibody and anti-BRI3 antibody) on the cells to the antibody, and will thereby release the cells from the bound antibody. According to this method, the cells can be recovered without remaining antibodies on the cells. A commercially available reagent, fro example, DETACHaBEAD (Dynal Biotech) may be used.

CD44, D6.1A and BRI3 are all transmembrane proteins, which have extracellular domains and are specifically expressed on the small hepatocytes. Mature hepatocytes do not express any of these proteins or express them only at an extremely low level. This can be confirmed at mRNA level by a method such as RT-PCR or Northern blot, or at protein level by Western blot or the like.

In one embodiment of the present invention, the antibody is linked to a magnetic bead, the antibody is contacted with cells, and the small hepatocytes are released with MACS. In another embodiment of the present invention, anti-CD44 antibody, anti-D6.1A antibody or anti-BRI3 antibody is biotinylated, and the small hepatocytes bound to anti-CD44 antibody, anti-D6.1A antibody or anti-BR13 are isolated by using a carrier linked to avidin, for example, magnetic beads linked to avidin. In a further embodiment of the present invention, an antibody (secondary antibody) which binds to at least one of anti-CD44 antibody, anti-D6.1A antibody and anti-BRI3 antibody (primary antibody) is labeled with biotin, and the small hepatocytes are isolated as an immunocomplex containing small hepatocytes - (anti-CD44 antibody, anti-D6.1A antibody and/or anti-BR13 antibody (primary antibody)) - biotin-labeled antibody (secondary antibody) by using an avidin-linked carrier such as avidin-linked magnetic beads.

In another embodiment of the present inventions, small hepatocytes are isolated by using flow cytometry. For example, anti-CD44 antibody, anti-D6.1A antibody and/or anti-BRI3 antibody are directly labeled with fluorochrome and these antibodies are contacted with cells. The small hepatocytes are isolated by sorting them with FACS. Additionally, anti-CD44 antibody, anti-D6.1A antibody and/or anti-BRI3 may be biotinylated and the small hepatocytes may be isolated similarly by using FACS with avidin labeled with fluorochrome. In another embodiment of the present invention where flow cytometry is used, an antibody (secondary antibody) which binds to at least one of anti-CD44 antibody, anti-D6.1A antibody and anti-BR13 antibody (primary antibody) is labeled with biotin, and the small hepatocytes are isolated as an immunocomplex containing small hepatocytes - (anti-CD44 antibody, anti-D6.1A antibody and/or anti-BRI3 antibody (primary antibody)) - biotin-labeled antibody (secondary antibody) similarly by using FACS with fluorochrome-labeled avidin.

Particularly when sorting is conducted with FACS, a cell that expresses one, two or all of CD44, D6.1A and BRI3 can be isolated individually by labeling these three antibodies with different dyes, respectively. Additionally, when FACS is used, a combination of a biotinylated antibody and a fluorochrome may be used only for one antibody.

When small hepatocytes are separated by using MACS, a column is filled with a magnetic bead-antibody and a cell population containing small hepatocytes, the cells bound to the antibody is fixed by using a magnet, the column is washed with a buffer (for example, 0.5 % bovine serum albumin/2 mM EDTA/PBS) of 10- to 20-fold volume of the column to remove cells which did not bound to the antibody, and then the small hepatocytes bound to the antibody may be recovered by removing the magnet. The magnetic beads may optionally be cleaved from the antibody with a commercially available reagent or by enzymatically digesting the Fc portions of the antibody.

Various markers may be used to confirm that such isolated small hepatocytes have some of the functions of hepatocytes. Such markers include, besides albumin which is secreted in the medium, transferrin, production of urea, amount of glycogen, cytochrome P450 and the like. These markers may be confirmed by ELISA, Western blot analysis or RT-PCR and the like for the medium or the cell extract, or by directly immunostaining the cells. The small hepatocytes can be distinguished from other cells in that they exhibit the markers of mature hepatocytes such as albumin, CK8, CK18, glycogen or peroxisome(s) but they do not exhibit the markers of biliary epithelial cells such as CK7 or CK19. For example, oval cells, which are known to be a kind of hepatic stem cells, exhibits the markers of biliary epithelial cells such as CK7 and CK19 but they do not exhibit the markers of mature hepatocytes such as glycogen or peroxisome(s). Additionally, it can be confirmed that other markers for non-parenchymal cells (ED1/2 (a marker for Kupffer cells), SE-1 (a marker for sinusoidal endothelial cells), desmin (a marker for Ito cells), vimentin (a marker for liver epithelial cells)) are negative.

The efficiently isolated small hepatocytes as such may be cultured in analogy to the culture of the fraction which is obtained from a liver and contains a large amount of small hepatocytes by using the medium and culture container as described above. Additionally, such efficiently isolated, cultured and maintained small hepatocytes can be cryopreserved according to the method described in WO 01/092481 or matured to a liver tissue according to the method described in WO 02/088332, and are also used to estimate drug functions by using the matured liver tissue.

As a culture container used for culturing the small hepatocytes isolated according to the present invention for cryopreservation or for use in estimating drug functions, a hyaluronic acid-attached culture dish may be used, but a conventional culture dish used for cell culture may be also used. Generally, collagen-coated dishes are used for the culture of adherent cells. For example, the culture dishes of various sizes which are coated by collagen derived from bovine dermis or rat tail are commercially available. It is also possible to prepare such dishes, if necessary. It is preferable to use dishes which are not coated by collagen to form small hepatocyte colonies which are suitable to generate a liver tissue, because the cells can be dissociated from the dishes in the mild condition more easily when the extracellular matrix is less and the small hepatocytes are likely to be preferentially dissociated from the dishes. A conventional 5% CO₂ incubator may be used for the culture. The range of CO₂ concentration and the temperature are not essential as long as they are within the range which is acceptable for conventional cultured cells.

### Example

### Example 1. Preparation of a fraction enriched with small hepatocytes

Adult rats (8 to 10 weeks old) received D-galactosamine (Across) by an intraperitoneal injection at a concentration of 75 mg/200 µl PBS/100 g body weight. At day 4 of post-injection, cells were dissociated from the liver according to the method of Seglen. Hanks' solution was perfused for about 4 minutes at a flow rate of 40 ml/min, and then Hanks' solution containing 0.02 % collagenase (Yakult Co.) was perfused for 10 minutes at a flow rate of 20 ml/min. The hepatocytes were dropped into a beaker from the digested liver according to an ordinary method. The cell suspension was passed through a 250 µm- and then 70 µm-mesh filter and centrifuged for one minute at 50 x g. The supernatant was collected and centrifuged twice for one minute at 50 x g. The supernatant was collected and centrifuged for 5 minutes at 50 x g. The precipitated cells were washed with a medium (Leibovitz L-15 + 10% fetal bovine serum + 10⁻⁷ M dexamethasone + 0.5 mg/ml insulin + antibiotics) and were centrifuged for 5 minutes at 50 x g. The similar procedure was repeated and the suspension was centrifuged again for 5 minutes at 150 x g. The similar procedure was repeated and the suspension was centrifuged again for 5 minutes at 50 x g. The precipitated cells were suspended in the fresh medium and the number of viable cells was counted to adjust the cell density to 1.0 x 10⁴ to 5 x 10⁵ cells/ml.

### Example 2. Analysis of expression pattern of CD44, D6.1A and BRI3 on small hepatocytes

### 1) Time course of expression pattern of CD44, D6.1A and BRI3

Total RNA was prepared from small hepatocytes immediately after preparation or after starting culture, and the expression of CD44 was determined by RT-PCR method. GPDH (glycerol-3-phosphate dehydrogenase) was used as a positive control. The following medium was used for culturing the small hepatocytes:

| Dulbecco's modified Eagle's medium (GIBCO Laboratories) | |
|---|---|
| + 20 mM HEPES | (Dojindo) |
| + 25 mM NaHCO₃ | (Katayama Chemical Co.) |
| + 30 mg/l proline | (Sigma Chemical Co.) |
| + 0.5 mg/l insulin | (Sigma Chemical Co.) |
| + 10⁻⁷ M dexamethasone | (Sigma Chemical Co.) |
| + 10% FBS | (Hyclone Laboratories, Inc.) |
| + 10 mM nicotinamide | (Katayama Chemical Co.) |
| + 1 mM L-ascorbic acid 2-phosphate | (Wako Pure Chemical Inc.) |
| + 10 µg/l EGF | (Collaborative Research Inc.) |
| + antibiotics | |
| 1% dimethylsulfoxide (DMSO) | (Aldrich) |

| | |
|---|---|
| (DMSO was added to the culture medium on and after day 4 of medium exchange.) | |

Total RNA was prepared from about 1.0-1.5 x 10⁵ small hepatocytes by using ISOGEN (NIPPON GENE Co.). The cells were lysed according to the protocol recommended by the manufacturer, chloroform was added to the lysate and mixed well, and then the lysate was centrifuged at 4°C for 15 minutes at 10000 x g. The upper layer was removed and isopropanol was added to it, centrifuged at 4°C for 15 minutes at 10000 x g, and the resulting precipitate was washed with 70% ethanol and dissolved in DEPC-water. RNA from mature hepatocytes was also extracted from cells immediately after perfusion by using the similar method. Ten nanogram of the total RNA was used for RT-PCR. The primer used was as follows.

Primers for CD44:
5'-cccgaattcatggacaaggtttggtggca-3' (SEQ ID NO:1)
5'-cccgaattcctacaccccaatcttcatat-3' (SEQ ID NO:2)

Primers for D6.1A
5'-cccgaattcatggcaggtgtcagtggctg-3' (SEQ ID NO:3)
5'-cccgaattctcatttgcttccaatttggc-3' (SEQ ID NO:4)

Primers for BRI3
5'-cccgaattcatggtgaagatcagtttcca-3' (SEQ ID NO:5)
5'-cccgaattctcacaccaccccacagatga-3' (SEQ ID NO:6)

The following primers were used for GPDH:
5'-accacagtccatgccatcac-3' (SEQ ID NO:7)
5'-tccaccaccctgttgctgta-3' (SEQ ID NO:8)
The condition for PCR was as follows : 95°C-15 sec., 60°C-30 sec., 68°C-1 min., cycles=25

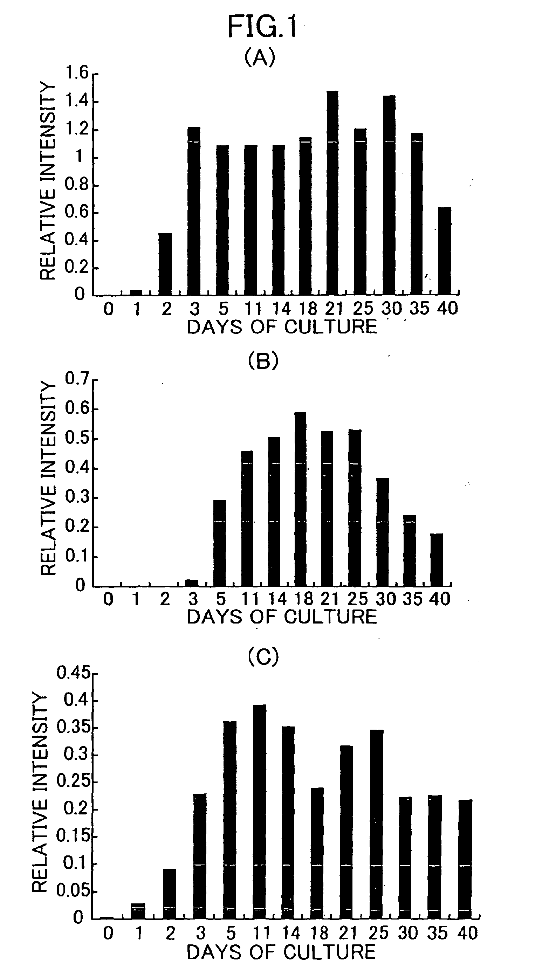

The results revealed that the significant expression of CD44 was observed at about 2 to 3 days after starting the primary culture and that the expression continued to increase up to about a maximum 5 days after starting the culture and the expression level of CD44 was maintained in the proliferating small hepatocytes. Regarding D6.1A, the expression was observed from about day 3 of the primary culture and reached to nearly a maximum at day 18 of the primary culture. Regarding BRI3, the expression is significant from about day 2 of the primary culture and reached to nearly a maximum at day 5 to 11 post-culture. The PCR products were subjected to electrophoresis and each band was detected by a densitometer, and the intensity of CD44 band was divided by the intensity of GPDH band to calculate the relative intensity (Figures 1A-1C).

### 2) Comparison of the expression patterns of CD44, D6.1A and BRI3 in small hepatocytes and in mature hepatocytes

The cells (about 1.0-1.5 x 10⁸ cells) which had been cryopreserved according to the method described in WO 01/92481 were thawed according to a conventional procedure and cultured for two weeks. Total RNA was extracted from the cultured cells by using the similar procedure described in 1). RNA from mature hepatocytes was extracted from the cells immediately after the perfusion by using the similar method.

Ten nanogram of the obtained total RNA was used for determining the relative expression level of CD44, D6.1A and BRI3 by using RT-PCR similarly as described in 1), except that the number of cycle was 35. The PCR products were subjected to electrophoresis and each band was detected by a densitometer, and the respective intensity of CD44, D6.1A and BRI3 bands was divided by the intensity of GPDH band to compare the expression of these antigens in small hepatocytes with that in mature hepatocytes.

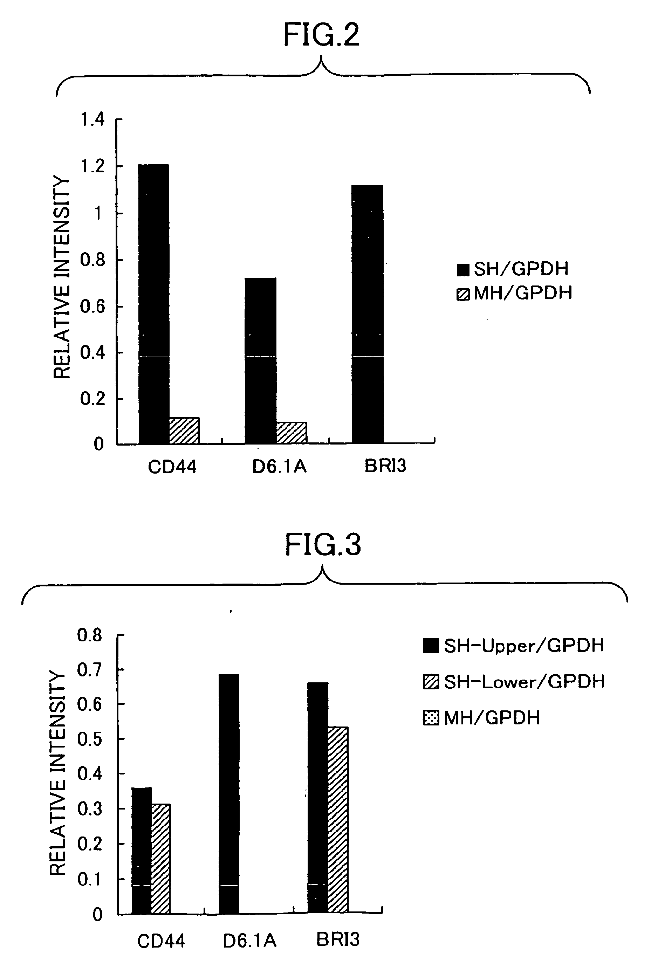

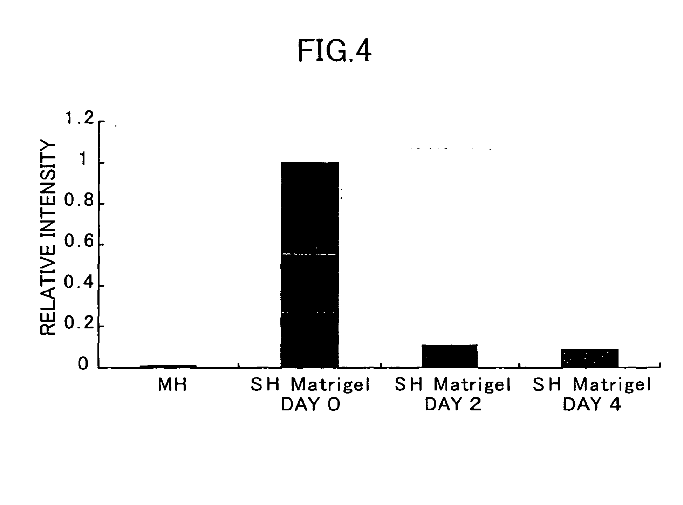

The results revealed that the expression of CD44, D6.1A and BR13 were consistently and extremely low in the mature hepatocytes (Figures 2 and 3) ant that the expression of these antigens in cells which were maturing from small hepatocytes decrease gradually as the cells matured (Figures 1 and 4).

Similar results were obtained with Northern blot. Again, in this case GPDH was used as a housekeeping gene. The resulting bands were read out by a densitometer, and the respective intensity of CD44, D6.1A and BRI3 band was divided by the intensity of GPDH band to compare the expression of these antigens in small hepatocytes and in mature hepatocytes.

Twenty microgram of thus obtained RNA was subjected to electrophoresis and Northern blotting. The probes used were the full length coding region for CD44, D6.1A or BRI3. The coding regions for CD44, D6.1A and BRI3 were shown in SEQ ID Nos:9, 10 and 11, respectively. The probes were labeled by using Alkphos Direct Labeling Kit (Amersham Biosciences).

The results revealed two bands for CD44 and BRI3, and one band for D6.1A. The resulting bands were read out by a densitometer, and the intensity of each band was divided by the intensity of GPDH band to compare the expression of these antigens in small hepatocytes and in mature hepatocytes. The results were similar to those obtained by RT-PCR (Figure 3).

Additionally, the change in CD44 expression was determined by Northern blot after inducing the maturation of small hepatocytes by adding Matrigel into the cultured small hepatocytes according to the method described in WO 02/088332. It has been known that the maturation started at day 2 after adding Matrigel. It was shown that the expression of CD44 decreased concurrently with the maturation (Figure 4).

These results demonstrate that CD44, D6,1A and BRI3 are specific to small hepatocytes and also suggest the possibility that the expressions of CD44, D6.1A and BRI3 are induced by a damage of preparing a cell fraction from a liver and that small hepatocytes which has potentially existed in the cell population composing a liver or precursors thereof elicited their inherent properties.

### Example 3. Isolation of proliferative hepatocytes with anti-CD44 antibody

The supernatant obtained after three times centrifugation of 50 x g as described in the aforementioned "1. Preparation of a fraction enriched with small hepatocytes" was collected and centrifuged for 5 minutes at 150 x g. The precipitated cells were suspended in a buffer (phosphate buffered saline (PBS) + 2mM EDTA (SIGMA Chemical Co.) + 0.5 % bovine serum albumin (Serologicals Proteins Inc.)).

The buffer was added to the cell at 80-99 ml per 10⁷ cells to suspend the cells, and 1-20 ml of mouse anti-rat CD44 antibody (PharMingen) was added to the suspension for 5-10 minutes at 4-8°C. The buffer of 10- to 20-fold volume was added to the suspension and which was centrifuged for 10 minutes at 300 x g. The precipitate following the centrifugation was resuspended in the buffer and centrifuged again for 10 minutes at 300 x g. The precipitate was suspended in a buffer (80 ml per 1 x 10⁷ cells) and 20ml of MACS anti-mouse IgG magnetic antibody (Miltenyi Biotec) was added , which was incubated for 15 minutes at 4-8°C. The buffer of 10- to 20-fold volume was added to the suspension, which was centrifuged for 10 minutes at 200 x g. Following the centrifugation the precipitate was suspended in 500 ml per 1 x 10⁸ cells (when the cells was les than 1 x 10⁸, uniformly 500 ml). MACS column (Miltenyi Biotec) was mounted on a multi-stand and 500 ml of the buffer was run through the column. Before the column dried the cell suspension was passed through the column and then 500 ml of the buffer was passed through the column 3 times to wash the column. The passed eluent up to this stage was designated as CD44 negative fraction. The column was removed from a magnet and 1 ml of the buffer was injected in the column and CD44 positive fraction was obtained by strongly extruding it with a syringe.

The obtained CD44-positive cells were morphologically equivalent with small hepatocytes and were capable of proliferating and forming colonies. The cells which formed the colonies secreted albumin and were negative for markers for non-parenchymal cells (ED1/2 (a marker for Kupffer cells), SE-1 (a marker for sinusoidal endothelial cells), desmin (a marker for Ito cells) and vimentin (a marker for liver epithelial cells)) as analyzed by immunostaining. Thus, the obtained cells were confirmed to be the small hepatocytes.

### Example 4. Isolation of proliferative hepatocytes with anti-D6.1 A antibody

D6.1A-positive cells were obtained by using rabbit anti-rat D6.1A antibody, similarly to Example 3. The obtained D6.1A positive cells were morphologically equivalent with small hepatocytes.

For the rabbit anti-rat D6.1A antibody, Immuno-Biological Laboratories Co., Ltd. was requested to produce the antibody by using FGAADWGKNFPDAKESC (DEQ ID NO:12) as an immunogen.

### Example 5. Isolation of proliferative hepatocytes with anti-BR13 antibody

BR13-positive cells were obtained by using rabbit anti-rat BR13 antibody, similarly to Example 3.

The Immuno-Biological Laboratories Co., Ltd. was requested to produce the rabbit anti-rat BRI by using LTPAREERPPRHRSRKGGSV (DEQ ID NO:13) as an immunogen.

The obtained BRI3-positive cells were morphologically equivalent with small hepatocytes and were capable of proliferating and forming colonies. The cells which formed the colonies were CK8-positive cells. Additionally the cells were negative for markers for non-parenchymal cells (ED1/2 (a marker for Kupffer cells, SE-1 (a marker for sinusoidal endothelial cells), desmin (a marker for Ito cells) and vimentin (a marker for liver epithelial cells)) as analyzed by immunostaining. Thus, the obtained cells were confirmed to be the small hepatocytes.

According to the present invention, small hepatocytes can be efficiently isolated from a liver of a mammalian including human being. When a mixture of cells was obtained from a liver of a mammalian including human being, various types of cells may contained in the mixture, but the small hepatocytes can be easily and efficiently isolated from the mixture according to the present invention.

### References

1. International Publication WO 01/92481
2. International Publication WO 02/088332
3. Japanese Patent No. 3211941
4. Laid Open Publication of Japanese Patent Application (JP-Kokai) No. 2002-078481
5. JP-Kokai No.09-313172
6. JP-Kokai No.08-112092
7. JP-Kokai No. 2002-045087
8. Mitaka T. et al., Hepatology 16, 440-447 (1992)
9. Mitaka T., Sato F., Mizuguchi T. et al., Hepatology 29, 111-135 (1999)

## Claims

1. A method of isolating small hepatocytes, comprising the steps of
i) contacting a cell suspension containing cells from the liver of a mammal with one or more antibodies selected from the group consisting of an antibody against CD44 (anti-CD44 antibody), an antibody against D6.1A (anti-D6.1A antibody) and an antibody against BR13 (anti-BR13 antibody) to form an immunocomplex containing the small hepatocytes which were contained in the cell suspension and the one or more antibodies;
and,
ii) recovering the immunocomplex containing the small hepatocytes.

2. The method according to claim 1, comprising the steps of
i) contacting a cell suspension containing cells from the liver of a mammal with one or more antibodies selected from the group consisting of anti-CD44 antibody, anti-D6.1A antibody and anti-BRI3 antibody to form a first immunocomplex containing the small hepatocytes which were contained in the cell suspension and the one or more antibody;
ii) contacting the first immunocomplex with one or more antibodies which bind to at least one of the antibodies used in step i) selected from the group consisting of anti-CD44 antibody, anti-D6.1A antibody and anti-BRI3 antibody to form a second immunocomplex; and,
iii) recovering the second immunocomplex containing the small hepatocytes.

3. The method according to claim 1, wherein the one or more antibodies selected from the group consisting of anti-CD44 antibody, anti-D6.1A antibody and anti-BRI3 antibody is labeled with biotin and the step of recovering the second immunocomplex containing the small hepatocytes comprises contacting the immunocomplex with a carrier linked to avidin and/or with avidin labeled with a fluorochrome.

4. A method of isolating small hepatocytes, comprising the steps of
i) contacting one or more of antibodies selected from the group consisting of anti-CD44 antibody, anti-D6.1A antibody, anti-BRI3 antibody with one or more antibody which binds to at least one or more antibodies selected from the group consisting of anti-CD44 antibody, anti-D6.1A antibody, anti-BR13 antibody to form a first immunocomplex;
ii) contacting the first immunocomplex with a cell suspension containing cells from the liver of a mammal to form a second immunocomplex; and
iii) recovering the second immunocomplex containing the small hepatocytes.

5. A method according to claim 1 wherein the one or more antibodies selected from the group consisting of anti-CD44 antibody, anti-D6.1A antibody and anti-BRI3 antibody is linked to a carrier.

6. A method according to claim 2, wherein the one or more antibodies which bind to at least one of the anti-CD44 antibody, anti-D6.1A antibody,and anti-BRI3 antibody used in step i) is linked to a carrier.

7. A method according to claim 4, wherein the one or more antibodies which bind to at least one of the one or more of antibodies selected from the group consisting of anti-CD44 antibody, anti-D6.1A antibody and anti-BRI3 is linked to a carrier.

8. The method according to any one of claims 3 and 5 to 7, wherein the carrier is a magnetic bead.

9. The method according to any one of claims 1 to 7, wherein the step of recovering the immunocomplex is conducted by flow cytometry.

## Patentansprüche

1. Verfahren zum Isolieren kleiner Hepatozyten, umfassend die Schritte
i) in Kontakt bringen einer Zellsuspension, welche Zellen aus der Leber eines Säugetiers enthält, mit einem oder mehreren Antikörpern ausgewählt aus der Gruppe bestehend aus einem Antikörper gegen CD44 (anti-CD44 Antikörper), einem Antikörper gegen D6.1A (anti-D6.1A Antikörper) und einem Antikörper gegen BRI3 (anti-BRI3 Antikörper) um einen Immunkomplex zu bilden, welcher die kleinen Hepatozyten, die in der Zellsuspension enthalten waren und den einen oder die mehreren Antikörper enthält; und
ii) Wiedergewinnung des Immunkomplexes, welcher die kleinen Hepatozyten enthält.

2. Verfahren nach Anspruch 1, umfassend die Schritte
i) in Kontakt bringen einer Zellsuspension, welche Zellen aus der Leber eines Säugetiers enthält, mit einem oder mehreren Antikörpern ausgewählt aus der Gruppe bestehend aus anti-CD44 Antikörper, anti-D6.1A Antikörper und anti-BRI3 Antikörper um einen ersten Immunkomplex zu bilden, welcher die kleinen Hepatozyten, die in der Zellsuspension enthalten waren und den einen oder die mehreren Antikörper enthält;
ii) in Kontakt bringen des ersten Immunkomplexes mit einem oder mehreren Antikörpern, welche(r) an mindestens einen der Antikörper, die in Schritt i) verwendet wurden, ausgewählt aus der Gruppe bestehend aus anti-CD44 Antikörper, anti-D6.1A Antikörper und anti-BRI3 Antikörper bindet/binden um einen zweiten Immunkomplex zu bilden; und
iii) Wiedergewinnung des zweiten Immunkomplexes, welcher die kleinen Hepatozyten enthält.

3. Verfahren nach Anspruch 1, wobei der eine oder die mehreren Antikörper ausgewählt aus der Gruppe bestehend aus anti-CD44 Antikörper, anti-D6.1A Antikörper und anti-BRI3 Antikörper mit Biotin markiert ist/sind und der Schritt der Wiedergewinnung des zweiten Immunkomplexes, welcher die kleinen Hepatozyten enthält, das in Kontakt bringen des Immunkomplexes mit einem Träger, der mit Avidin verknüpft ist und/oder mit Avidin, das mit einem Fluorochrom markiert ist, umfasst.

4. Verfahren zum Isolieren kleiner Hepatozyten, umfassend die Schritte
i) in Kontakt bringen des einen oder der mehreren Antikörper ausgewählt aus der Gruppe bestehend aus anti-CD44 Antikörper, anti-D6.1A Antikörper, anti-BRI3 Antikörper mit einem oder mehreren Antikörpern, welche(r) an mindestens einen der Antikörper ausgewählt aus der Gruppe bestehend aus anti-CD44 Antikörper, anti-D6.1A Antikörper, anti-BRI3 Antikörper bindet/binden um einen ersten Immunkomplex zu bilden;
ii) in Kontakt bringen des ersten Immunkomplexes mit einer Zellsuspension, welche Zellen aus der Leber eines Säugetiers enthält um einen zweiten Immunkomplex zu bilden; und
iii) Wiedergewinnung des zweiten Immunkomplexes, welcher die kleinen Hepatozyten enthält.

5. Verfahren nach Anspruch 1, wobei der eine oder die mehreren Antikörper ausgewählt aus der Gruppe bestehend aus anti-CD44 Antikörper, anti-D6.1A Antikörper und anti-BRI3 Antikörper mit einem Träger verknüpft ist/sind.

6. Verfahren nach Anspruch 2, wobei der eine oder die mehreren Antikörper, welche(r) an mindestens einen der Antikörper anti-CD44 Antikörper, anti-D6.1A Antikörper und anti-BRI3 Antikörper, die in Schritt i) verwendet werden, bindet/binden, mit einem Träger verknüpft ist/sind.

7. Verfahren nach Anspruch 4, wobei der eine oder die mehreren Antikörper, welche(r) an mindestens einen der Antikörper ausgewählt aus der Gruppe bestehend aus anti-CD44 Antikörper, anti-D6.1A Antikörper und anti-BRI3 Antikörper, bindet/binden, mit einem Träger verknüpft ist/sind.

8. Verfahren nach einem der Ansprüche 3 und 5 bis 7, wobei der Träger ein magnetisches Kügelchen ist.

9. Verfahren nach einem der Ansprüche 1 bis 7, wobei der Schritt der Wiedergewinnung des Immunkomplexes mittels Durchflusszytometrie durchgeführt wird.

## Revendications

1. Procédé d'isolement de petits hépatocytes, comprenant les étapes suivantes :
i) la mise en contact d'une suspension cellulaire contenant des cellules provenant du foie d'un mammifère avec un ou plusieurs anticorps choisis dans le groupe constitué par un anticorps dirigé contre CD44 (anticorps anti-CD44), un anticorps dirigé contre D6.1A (anticorps anti-D6.1A) et un anticorps dirigé contre BRI3 (anticorps anti-BRI3) pour former un immunocomplexe contenant les petits hépatocytes qui étaient contenus dans la suspension cellulaire et les un ou plusieurs anticorps ; et
ii) la récupération de l'immunocomplexe contenant les petits hépatocytes.

2. Procédé selon la revendication 1, comprenant les étapes suivantes :
i) la mise en contact d'une suspension cellulaire contenant des cellules provenant du foie d'un mammifère avec un ou plusieurs anticorps choisis dans le groupe constitué par l'anticorps anti-CD44, l'anticorps anti-D6.1A et l'anticorps anti-BRI3 pour former un premier immunocomplexe contenant les petits hépatocytes qui étaient contenus dans la suspension cellulaire et les un ou plusieurs anticorps ;
ii) la mise en contact du premier immunocomplexe avec un ou plusieurs anticorps qui se lient à au moins l'un des anticorps utilisés dans l'étape i) choisis dans le groupe constitué par l'anticorps anti-CD44, l'anticorps anti-D6.1A et l'anticorps anti-BRI3 pour former un second immunocomplexe ; et,
iii) la récupération du second immunocomplexe contenant les petits hépatocytes.

3. Procédé selon la revendication 1, dans lequel les un ou plusieurs anticorps choisis dans le groupe constitué par l'anticorps anti-CD44, l'anticorps anti-D6.1A et l'anticorps anti-BRI3 sont marqués avec de la biotine et l'étape de récupération du second immunocomplexe contenant les petits hépatocytes comprend la mise en contact de l'immunocomplexe avec un support lié à de l'avidine et/ou avec de l'avidine marquée avec un fluorochrome.

4. Procédé d'isolement de petits hépatocytes, comprenant les étapes suivantes :
i) la mise en contact d'un ou de plusieurs des anticorps choisis dans le groupe constitué par l'anticorps anti-CD44, l'anticorps anti-D6.1A et l'anticorps anti-BRI3 avec un ou plusieurs anticorps qui se lient à au moins un ou plusieurs anticorps choisis dans le groupe constitué par l'anticorps anti-CD44, l'anticorps anti-D6.1A et l'anticorps anti-BRI3 pour former un premier immunocomplexe ;
ii) la mise en contact du premier immunocomplexe avec une suspension cellulaire contenant des cellules provenant du foie d'un mammifère pour former un second immunocomplexe ; et
iii) la récupération du second immunocomplexe contenant les petits hépatocytes.

5. Procédé selon la revendication 1, dans lequel les un ou plusieurs anticorps choisis dans le groupe constitué par l'anticorps anti-CD44, l'anticorps anti-D6.1A et l'anticorps anti-BRI3 sont liés à un support.

6. Procédé selon la revendication 2, dans lequel les un ou plusieurs anticorps qui se lient à au moins l'un de l'anticorps anti-CD44, de l'anticorps anti-D6.1A et de l'anticorps anti-BRI3 utilisés dans l'étape i) sont liés à un support.

7. Procédé selon la revendication 4, dans lequel les un ou plusieurs anticorps qui se lient à au moins l'un des un ou plusieurs des anticorps choisis dans le groupe constitué par l'anticorps anti-CD44, l'anticorps anti-D6.1A et l'anticorps anti-BRI3, sont liés à un support.

8. Procédé selon l'une quelconque des revendications 3 et 5 et 7, dans lequel le support est une bille magnétique.

9. Procédé selon l'une quelconque des revendications 1 à 7, dans lequel l'étape de récupération de l'immunocomplexe est conduite par cytométrie en flux.
